# EUROPEAN PATENT APPLICATION

(11) **EP 3 805 257 A1**
(43) Date of publication of application: **14.04.2021**
(21) Application number: 19808332.1
(22) Date of filing: 23.05.2019
(51) Int. Cl.: C07K 14/62, C07K 1/36, C12P 21/00

(54) **METHOD FOR PREPARING PRECURSOR OF RECOMBINANT HUMAN INSULIN OR ANALOGUE THEREOF**

(30) Priority: 24.05.2018 CN 201810507928
(71) Applicant: Jiangsu Hengrui Medicine Co., Ltd., Lianyungang, Jiangsu 222047 (CN)
(72) Inventor: ZHAO, Liangliang, Lianyungang, Jiangsu 222047 (CN); HAN, Guangjie, Lianyungang, Jiangsu 222047 (CN); NIU, Ningning, Lianyungang, Jiangsu 222047 (CN); LI, Na, Lianyungang, Jiangsu 222047 (CN); LIU, Yanwei, Lianyungang, Jiangsu 222047 (CN); WANG, Hongwei, Lianyungang, Jiangsu 222047 (CN)
(74) Representative: dompatent von Kreisler Selting Werner - Partnerschaft von Patent- und Rechtsanwälten mbB
(86) International application number: PCT/CN2019/088083
(87) International publication number: WO 2019/223752

(57) **Abstract**

Disclosed is a method for preparing a precursor of a recombinant human insulin or an analogue thereof, comprising: a. bacterial fermentation, centrifuging a fermentation broth in a continuous flow to collect a supernatant; b. filtering the supernatant in step (a) by means of a hollow fiber membrane and collecting the filtrate; and c. purifying the filtrate in the step (b) by means of a chromatography column. The method has the advantages of streamlined steps, scalability, no use of organic solvents, high yield, etc. The purity of the insulin precursor can exceed 90%, the host cell protein removal rate exceeds 90%, and the exogenous DNA removal rate is 89% or greater, achieving less than 0.1 ng/mg.

## Description

The present application claims priority of Chinese patent application CN201810507928.7 filed on May 24th, 2018, the content of which is incorporated herein by reference in its entirety.

### Technical Field

The invention relates to the field of protein and polypeptide drugs, in particular to a method for preparing a precursor of recombinant human insulin or an analogue thereof, thereby preparing an insulin analogue drug for treating diabetes-related disease.

### Backgroud

Diabetes is a common metabolic and endocrine disease, and it is the third most serious life-threatening disease after cancer and cardiovascular and cerebrovascular diseases. In the treatment of diabetes, insulin is the first line drug. The rapid growth in the number of diabetic patients makes the demand for insulin increase rapidly. With the rapid development of biotechnology, it has become a mainstream method for the industry to express human insulin and analogues thereof by using microbial platforms and genetic engineering.

There are a variety of insulin analogues or their derivatives. WO2005012347 disclosed an insulin analogue in which the amino acid at position B30 is deleted and a glutamic acid and a long-chain fatty acid are linked at position B29. WO2007074133 and WO2011141407 respectively disclosed preparations of the above insulin analogues and preparation method thereof; WO9507931 disclosed an insulin analogue, a formulation and preparation method thereof, wherein the amino acid at position B30 is deleted and a tetradecyl side chain is linked at position B29 of the insulin analogue; WO2018024186 disclosed the structure and biological activity of an acylated derivative of a human insulin analogue.

With regard to the preparation of insulin analogues or derivatives thereof, in 1982, Genetech and Lilly cooperated in the production of insulin by using *E. coli* system. Insulin precursor, which was existed in *E. coli* cells in the form of inclusion body, was released after cell lysis. Insulin precursors with the correct structure were obtained after renaturation from cell lysis. CN1043719A disclosed a use of a yeast system for the production of insulin, wherein the insulin precursor can be secreted directly into the fermentation broth, and the supernatant after removal of bacteria by centrifugation is pass through a chromatography column, thereby isolating the precursor by freeze drying. CN106282274A disclosed a method for preparing insulin precursor protein by high-density fermentation of *P. pastoris* and performing solid-liquid separation on fermentation broth by centrifutation. US2014057318A disclosed a method for clarifying the fermentation broth of insulin precursor by using a hollow fiber membrane system. CN1290299A disclosed a method for capturing insulin precursor by adsorption with a macroporous resin followed by elution with an organic solvent. CN105153294A disclosed a method that Capto MMC series fillers can be used for directed loading to obtain insulin precursor which can be directly used for enzyme digestion.

Clarification of the fermentation broth is the first step in the preparation of insulin precursor secreted and expressed by yeast, which aims to separate bacteria and protein and obtain fermentation supernatant containing insulin precursor. Conventional methods for separating yeast solid and liquid include plate and frame filter press, clarification and separation using hollow fiber membrane, centrifugation, flocculation and sedimentation, expanded bed chromatography and so on. After the fermentation broth is clarified, a clear liquid containing insulin precursor is obtained. The clear liquid usually needs to undergo a capture process before enzymatic digestion. Because of the high conductivity, the clear liquid needs to be diluted before ion exchange chromatography.

There are many problems in the existing methods for preparing precursors of recombinant human insulin or analogues thereof, including: high cost, time consuming, complicated operation, increased explosion-proof requirements of the production workshop due to using organic solvents, demand for expanding the area of the storage tank due to the excessive volume of the clear liquid, the inability of the obtained insulin precursor being direct digested, and the low recovery rate of insulin precursor product, etc. In view of the above problems, there is an urgent need for an improved method for preparing precursors of recombinant human insulin or analogues thereof with high-efficiency, high-yield, low-cost, and easy to scale-up production.

### Summary of the invention

The present invention organically combines continuous flow centrifugation and hollow fiber microfiltration to prepare precursors of recombinant human insulin or analogues thereof, which is suitable for large-scale industrial production of insulin precursors and insulin.

Some embodiments provided a method for preparing a precursor of recombinant human insulin or an analogue thereof, comprising the following steps:
a. performing continuous flow centrifugation on obtained fermentation broth after the fermention of bacteria, and collecting a light liquid;
b. filtering the light liquid in step a by hollow fiber membrane filtration and collecting obtained filtrate;
c. purifying the filtrate in the step b by chromatography column.

In some embodiments, after continuous flow centrifugation, a light liquid with bacterial content of less than 1% can be obtained, and an insulin precursor with a purity of more than 90% can be obtained after chromatography.

In some embodiments, the hollow fiber membrane in step b preferably has a pore size of 0.22 µm or 0.45 µm. In at least one embodiment, the hollow fiber membrane has a pore size of 0.22 µm.

In some embodiments, the hollow fiber membrane filtration in step b is a cyclical tangential flow filtration, the hollow fiber membrane has a molecular weight cut-off of 500-1000 KDa. In at least one embodiment, the hollow fiber membrane has a molecular weight cut-off of 750 KDa.

In some embodiments, the method comprises the following steps:
a. performing continuous flow centrifugation on obtained fermentation broth after the fermentation of the bacteria, and collecting a light liquid and a heavy liquid respectively; diluting the heavy liquid at least once and then performing centrifugation again to obtain another light liquid, and pooling two obtained light liquids into one;
b. filtering the light liquid obtained in step a by a cyclical tangential flow filtration using a hollow fiber membrane and collecting the filtrate;
c. purifying the filtrate in step b by chromatography column,
wherein the filler used in chromatography column is a composite filler comprising a cation exchange ligand and a hydrophobic ligand. In some specific embodiments, Eshmuno®HCX is selected as a chromatography medium of the composite filler.

In some embodiments, the cation exchange ligand is a strong cation exchange ligand.

In some embodiments, the described diluting adopts a diluent that is selected from an acidic solution or an alkaline solution, wherein the acidic solution is selected from an acetic acid-sodium acetate buffer and a citrate buffer, and the alkaline solution is selected from a trihydroxymethyl-aminomethane-hydrochloric acid (Tris-HCl) buffer, a phosphate buffer and a glycine sodium hydroxide buffer. In at least one embodiment, the acidic solution has a pH of 2.0-6.0, and the alkaline solution has a pH of 7.0-9.0. In at least one embodiment, the concentration of the acidic solution is 1 mM-100 mM, or 1mM - 50mM, or 10mM - 20mM, or is or is about 10mM, 20mM, 30mM, 40mM, 50mM, 60mM, 70mM, 80mM, 90mM or 100mM. In at least one embodiment, the concentration of the alkaline solution is 1 mM-100 mM , or 1mM-50mM, or 10mM-30mM, or is or is about 10mM, 20mM, 30mM, 40mM, 50mM, 60mM, 70mM, 80mM, 90mM or 100mM.

In some embodiments, the fermentation broth in step (a) is adjusted to a pH of 2.0-9.0, or 3.0-8.5, or any point between 4.0 and 8.0 before centrifugation. In at least one embodiment, the pH is or is about 2.0, 2.5, 3.0, 3.5, 4.0, 4.5, 5.0, 5.5, 6.0, 6.5, 7.0, 7.5, 8.0 or 8.5.

In some embodiments, the continuous flow centrifugation is performed by a disc centrifuge.

In some embodiments, the centrifugal force of continuous flow centrifugation is any value in the range of 8000g and 14000g, e.g., is or is about 8000, 8500, 9000, 10000, 11000, 12000, 13000 or 14000g.

In some embodiments, the method comprises following steps:
a. adjusting the fermentation broth to a pH of 3.0-8.0 after the fermention of bacteria, performing continuous flow centrifugation and collecting a light liquid;
b. filtering the light liquid in step a by a cyclical tangential flow filtration system using a hollow fiber membrane and collecting the filtrate;
c. purifying the filtrate in step b by chromatography column using a strong cation-hydrophobic exchange ligand;
wherein, step a further comprises following steps: collecting light liquid and heavy liquid, respectively, diluting the heavy liquid at least once and then performing centrifugation to obtain another light liquid, and pooling two light liquids into one; the diluting adopts a diluent that is selected from an acetic acid-sodium acetate buffer with a pH of 3.0-5.0 or a Tris-HCl buffer with a pH of 7.0-9.0. The hollow fiber membrane in step b can have a pore size of 0.22 µm.

In some specific embodiments, the pH of the acetic acid-sodium acetate diluent can be or is about 3.0, 4.0 or 5.0, and the pH of the Tris-HCl buffer can be or is about 7.0, 8.0 or 9.0.

In some specific embodiments, the purification in step (c) comprises steps of equilibration, loading, washing impurities and elution; the equilibration and washing use a solution of an acetic acid-sodium acetate buffer, and the elution uses a solution of a Tris-HCl buffer. In some specific embodiments, the concentration of the acetic acid-sodium acetate buffer is 1-50 mM, or 10-40 mM, or 20-30 mM, or is or is about 10 mM, 20 mM, 30 mM, 40 mM or 50 mM. The concentration of the Tris-HCl buffer is 10mM-150mM, or 30mM-100mM, or 50mM-70mM, or is or is about 20mM, 30mM, 40mM, 50mM, 60mM, 70mM, 80mM, 90mM, 100mM, 110mM, 120mM, 130mM, 140mM or 150mM. The solution used in the equilibration has a pH of 3.0-6.0, or 4.0-5.0; the solution used in the washing impurities has a pH of 5.0-7.0, or 5.5-6.0.

Some embodiments also provided a method for preparing a human recombinant insulin or an analogue thereof, wherein the method comprising: 1) expressing a precursor of human recombinant insulin or an analogue thereof by yeast; 2) purifying the precursor of human recombinant insulin or the analogue thereof according to the method as defined above; 3) performing enzymatic digestion on the precursor of human recombinant insulin or the analogue thereof to obtain the human recombinant insulin or the analogue thereof;

In some specific embodiments, the human recombinant insulin analogue is human insulin with B30 deletion.

Some embodiments provided a method for preparing an acylated insulin derivative, the insulin derivative can be an acylated insulin analog, wherein the method comprising: 1) expressing a precursor of human recombinant insulin by yeast; 2) purifying the precursor of human recombinant insulin according to the method as defined above; 3) performing enzymatic digestion on obtained human recombinant insulin; 4) conducting a substitution of an acylation group on the human recombinant insulin.

In some embodiments, the acylated substitution is substituted at the lysine at position B29, the acylated insulin analogue may be or may not be a human recombinant insulin with B30 deletion. In some specific embodiments, the substitution has a product of Lysine B29 (N^{ε}-(N^{α}-hexadecanedioic acid-L-lysine-N^{ε}-oxobutanoyl)) des(B30) human recombinant insulin.

In some specific embodiments, the fermentation broth with high cell content (such as 40%) is processed by continuous slagging using continuous flow centrifuge, which can quickly achieve the initial separation of cell and supernatant, and obtain a light liquid with cell content less than 1%. The light liquid is filtered by hollow fiber microfiltration to obtain a clear liquid containing insulin precursor with high clarity. Compared with single hollow fiber membrane filtration, the volume of the clear liquid is reduced by 70%; compared with single continuous flow centrifuge, the clarity of which has been significantly improved.

In some embodiments, the yield of insulin precursors and analogues thereof obtained according to the above method is higher than 60%, the purity is higher than 80%, the removal rate of host cell protein is higher than 80%, and the removal rate of exogenous DNA is higher than 80%. In some specific embodiments, the yield of precursors of insulin and its analogues obtained according to the above method is higher than 70%, the purity is higher than 90%, the removal rate of host cell protein is higher than 85%, and the removal rate of exogenous DNA is higher than 85%. In some specific embodiments, the yield of precursors of insulin and its analogues obtained according to the above method is higher than 80%, the purity is higher than 90%, the removal rate of host cell protein is higher than 90%, and the removal rate of exogenous DNA is higher than 88%. In some specific embodiments, the yield of precursors of insulin and its analogues obtained according to the above method is higher than or equal to 81%, the purity is higher than 93%, the host cell protein removal rate is higher than 95%, and the removal rate of exogenous DNA is higher than 89%. In some specific embodiments, the yield of precursors of insulin and its analogues obtained according to the above method is higher than or equal to 85%, the purity is higher than 93%, the removal rate of host cell protein is higher than is higher than 96%, and the removal rate of exogenous DNA is higher than 89%. In some specific embodiments, the yield of precursors of insulin and its analogues obtained according to the above method is higher than or equal to 93%, the purity is higher than 90%, the removal rate of host cell protein is higher than or equal to 91%, and the removal rate of exogenous DNA is higher than or equal to 89%.

### Definitions

The term 'insulin' as used herein refers to insulin hormones with well-known structure and properties, including human insulin hormones. Human insulin has two polypeptide chains, named A chain and B chain. A chain is a peptide of 21 amino acid and B chain is a peptide of 30 amino acid. The two chains are conjugated by disulfide bridges: the first bridge locates between the cysteine at position 7 of A chain and the cysteine at position 7 of B chain, and the second bridge locates between the cysteine at position 20 of A chain and the cysteine at position 19 of B chain. The third bridge locates between the cysteines at positions 6 and 11 of A chain.

The term "parent insulin" refers to the insulin before any modification is applied to its amino acid sequence.

The term "insulin analogue" refers to modified insulin in which one or more amino acid residues of insulin have been substituted by other amino acid residues and/or one or more amino acid residues have been deleted from insulin and/or one or more amino acids have been added and/or inserted into insulin. In some embodiments, the insulin analogues contains less than 8 modifications (substitutions, deletions, additions (including insertions) and any combination thereof) relative to the parent insulin, alternatively less than 7 modifications relative to the parent insulin, alternatively less than 6 modifications relative to the parent insulin, alternatively less than 5 modifications relative to the parent insulin, alternatively less than 4 modifications relative to the parent insulin, alternatively less than 3 modifications relative to the parent insulin, alternatively less than 2 modifications relative to the parent insulin. Examples of insulin analogues include AspB28 human insulin and des(B30) human insulin.

The term "insulin derivative" refers to a chemically modified parent insulin or an analogue thereof, wherein the modification is in the form of linked amide, carbohydrate, alkyl, acyl, ester, PEGylation, etc. An example is Lysine B29 (N^{ε}-(N^{α}-hexadecanedioic acid-L-lysine-N^{ε}-oxobutanoyl)) des(B30) human insulin.

Compared with the prior art, the present invention has the following advantages:
1. the combination of continuous flow centrifugation and hollow fiber microfiltration can achieve quick clarification of the fermentation broth. Compared with method of single hollow fiber membrane filtration, it significantly reduces the volume of the clarified liquid and the volume of the storage tank, reduces the building area of the plant, and significantly shortens the loading time of downstream chromatography, which is particularly suitable for industrial production of clarification of high-density fermentation broth of *Pichia pastoris*;
2. the clarified liquid has high clarity and can be directly loaded for chromatography, thus reducing subsequent processing;
3. one-step chromatography of the clarified liquid can remove the pigment and HCP and obtain a high-concentration of insulin precursor that can be directly digested by enzyme simultaneously. The utilization of higher-load and lower-priced fillers significantly reduces the production cost of the capture process;
4. The obtained insulin precursors or analogues thereof have high yield, high purity and high removal rate of impurities (including host cell proteins and exogenous DNA, etc.).

### Brief description of the drawings

Figure 1: HPLC detection chart of captured sample of insulin precursor sample prepared by the method of Example 9.
Figure 2: HPLC detection chart of captured sample of insulin precursor sample prepared by the method of Example 10.
Figure 3: HPLC detection chart of captured sample of insulin precursor sample prepared by the method of Example 11.

### Detailed description of the preferred embodiment

The following examples are used to further describe the present invention, but the scope of present invention is not limited thereto.

Materials: MaxCell hollow fiber column and tangential flow filtration system were purchased from General Electric (China) Medical Group; Eshmuno®HCX was purchased from Merck Millipore China.

*Pichia pastoris*: All of the conventional *Pichia pastoris* for fermentation in the art can be used herein, for example, the GS115 type *Pichia pastoris* (purchased from Invitrogen, USA) in CN106282274A can be used to ferment and express recombinant human insulin precursor.

Recombinant human insulin and precursors thereof: The recombinant human insulin and precursors thereof herein can be any type of recombinant human insulin and precursors thereof, such as recombinant human insulin Lysine B29 (N^{ε}-(N^{α}-hexadecanedioic acid-L-lysine-N^{ε}-oxobutanoyl)) des(B30) human insulin and precursors thereof.

Confirmation of the structure of human insulin: After purification of the recombinant human insulin precursors in the present invention, the target product can be digested with, for example, V8 protease, and the digested product can be analyzed by LC-MS. Recombinant human insulin: the recombinant human insulin herein can be any kind of recombinant human insulin, such as the recombinant human insulin Lysine B29 (N^{ε}-(N^{α}-hexadecanedioic acid-L-lysine-N^{ε}-oxobutanoyl)) des(B30) human insulin in PCT/CN2019/074146.

### Example 1 Centrifugal separation of fermentation broth

*Pichia pastoris* was used to ferment and express insulin precursor. A 50L of fermentation broth with 40% bacteria content was adjusted to pH 4.0 with acetic acid, and treated with an Alfa Laval flow centrifuge T20 with a centrifugal force of 10,000 g, a flow rate of 60L/h and a frequency of slag discharge once every 2 min. A 25L of light liquid and a 25L of heavy liquid were collected. The heavy liquid was diluted to 50L with 10 mM acetic acid-sodium acetate of pH 4.0 and centrifuged again, and another 25L of light liquid was collected, thus collecting a total of 50L light liquid. The total yield of insulin precursor was 90% and the bacterial content was less than 1%.

### Example 2 Centrifugal separation of fermentation broth

*Pichia pastoris* was used to ferment and express insulin precursor. A 50L of fermentation broth with 40% bacteria content was adjusted to pH 5.0 with Tris, and treated with an Alfa Laval flow centrifuge T20 with a centrifugal force of 10,000 g, a flow rate of 60 L/h and a frequency of slag discharge once every 2 min. A 25 L of light liquid and a 25 L of heavy liquid were collected. The heavy liquid was diluted to 50 L with 10 mM acetic acid-sodium acetate of pH 5.0 and centrifuged again, and another 25 L of light liquid was collected, thus collecting a total of 50 L light liquid. The total yield of the insulin precursor was 95% and the bacterial content was less than 1%.

### Example 3 Centrifugal separation of fermentation broth

*Pichia pastoris* was used to ferment and express insulin precursor. A 500L of fermentation broth with 34% bacteria content was adjusted to pH 5.0 with acetic acid, and treated with a continuous flow centrifuge with a centrifugal force of 10,000 g, a feeding speed of 1000 L/h and a light liquid outlet pressure of 5 bar. A 210 L of light liquid and a 290 L of heavy liquid were collected. The heavy liquid was diluted to 400 L with 10 mM acetic acid-sodium acetate of pH 5.0 and centrifuged again, and another 120 L of light liquid was collected, thus collecting a total of 330 L light liquid. The total yield of insulin precursor was 88% and the bacterial content was less than 1%.

### Example 4 Centrifugal separation of fermentation broth

*Pichia pastoris* was used to ferment and express insulin precursor. A 350L of fermentation broth with 41% bacteria content was adjusted to pH 3.0 with acetic acid, and treated with a continuous flow centrifuge with a centrifugal force of 10,000 g, a feeding speed of 800 L/h and a light liquid outlet pressure of 5 bar. A 110 L of light liquid and a 240 L of heavy liquid were collected. The heavy liquid was diluted to 350 L with 10 mM acetic acid-sodium acetate of pH 3.0 and centrifuged again, and another 120 L of light liquid was collected, thus collecting a total of 230 L light liquid. The total yield of insulin precursor was 85% and the bacterial content was less than 1%.

### Example 5 Centrifugal separation of fermentation broth

*Pichia pastoris* was used to ferment and express insulin precursor. A 350L of fermentation broth with 40% bacteria content was adjusted to pH 8.0 with 5 M NaOH solution, and treated with an Alfa Laval flow centrifuge T20 with a centrifugal force of 10,000 g, a flow rate of 60 L/h, a feeding speed of 800 L/h and a light liquid outlet pressure of 5 bar. A 130 L of light liquid and a 220 L of heavy liquid were collected. The heavy liquid was diluted to 350 L with 20 mM Tris-HCl of pH 8.0 and centrifuged again, and another 140 L of light liquid was collected, thus collecting a total of 270 L light liquid. The total yield of insulin precursor was 95% and the bacterial content was less than 1%.

### Example 6 Hollow fiber microfiltration

A 0.22 µm of hollow fiber column was washed cyclically with an equilibration solution (10 mM acetic acid-sodium acetate, pH 4.0), until the pH in the reflux end and the feed inlet are equal. 50 L of the light liquid obtained in Example 1 was subjected to hollow fiber microfiltration, with a transmembrane pressure of 15 psi. About 48 L of filtrate was collected from the permeate outlet, and the yield of insulin precursor was 95%.

### Example 7 Hollow fiber microfiltration

A 0.22 µm of hollow fiber column was washed cyclically with an equilibration solution (10 mM acetic acid-sodium acetate, pH 5.0), until the pH in the reflux end and the feed inlet are equal. 50 L of the light liquid obtained in Example 2 was subjected to hollow fiber microfiltration, with a transmembrane pressure of 15 psi. About 48 L of filtrate was collected from the permeate outlet, and the yield of insulin precursor was 90%.

### Example 8 Hollow fiber microfiltration

A 0.22 µm of hollow fiber column was washed cyclically with an equilibration solution (20 mM Tris-HCl pH 8.0), until the pH in the reflux end and the feed inlet are equal. 270 L of the light liquid obtained in Example 5 was subjected to hollow fiber microfiltration, with a transmembrane pressure of 15 psi. About 255 L of filtrate was collected from the permeate outlet, and the yield of insulin precursor was 93%.

### Example 9 Capture of insulin precursor

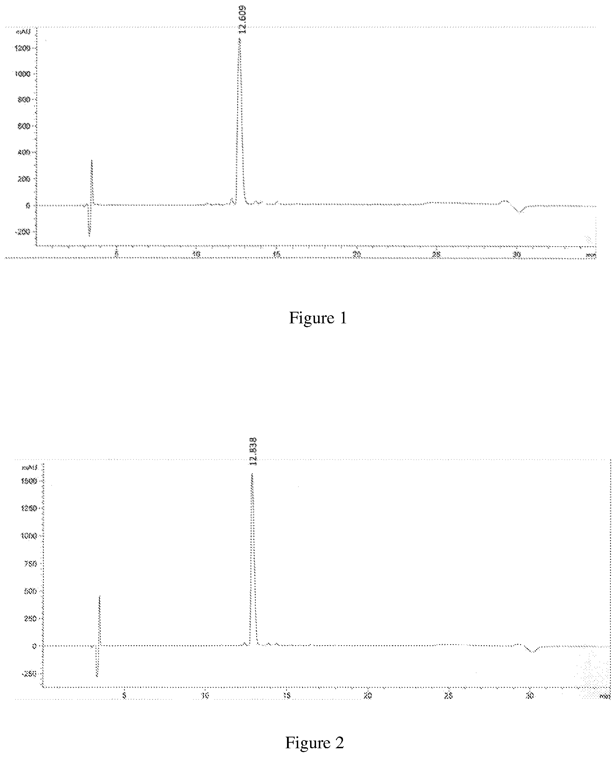

The filtrate obtained in Example 6 was subjected to cation chromatography, Eshmuno®HCX was used as composite chromatography medium, and the chromatography column was washed 3 times the column volume with an equilibration solution (10mM acetic acid-sodium acetate buffer, pH 4.0). The filtrate was directly loaded to the column with a sample volume of 60 g/L, then the column was washed 5 times the column volume with a washing solution (10mM acetic acid-sodium acetate buffer, pH 5.8) to remove portion of the pigment and HCP. Finally, an eluent (100 mM Tris-HCl, pH 8.0) was used for elution and the elution peak was collected to obtain insulin precursor with a purity of 93.1% and a yield of 81%. The results are shown in Figure 1. When detecting the host cell protein and exogenous DNA in the elution peak, it was found that the removal rate of the host cell protein and the exogenous DNA reached 95.6% and 89.6% respectively after one step of chromatography of the filtrate. The results are shown in Table 1.

### Example 10 Capture of insulin precursor

The filtrate obtained in Example 7 was subjected to cation chromatography, Eshmuno®HCX was used as composite chromatography medium, the chromatography column was washed 3 times the column volume with an equilibration solution (10mM acetic acid-sodium acetate buffer, pH 5.0). The filtrate was directly loaded to the column with a sample volume of 80 g/L, then the column was washed by 5 times the column volume with a washing solution (10mM acetic acid-sodium acetate buffer, pH 6.0) to remove portion of the pigment and HCP. Finally, an eluent (30 mM Tris-HCl, pH 8.5) was used for elution and the elution peak was collected to obtain insulin precursor with a purity of 93.5% and a yield of 85%. The results are shown in Figure 2. When detecting the host cell protein and exogenous DNA in the elution peak, it was found that the removal rate of the host cell protein and the exogenous DNA reached 96.8% and 89.8% respectively after one step of chromatography of the filtrate. The results are shown in Table 1.

### Example 11 Capture of insulin precursor

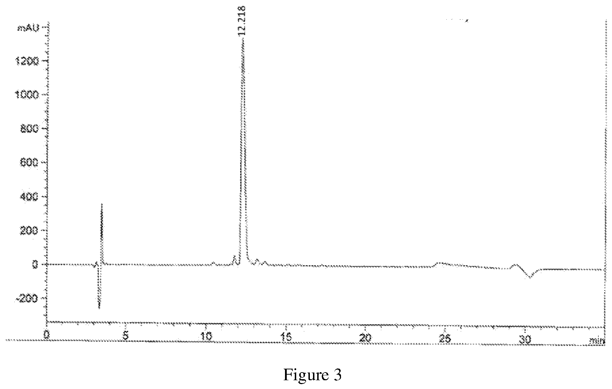

The filtrate obtained in Example 8 was adjusted to pH 5.0, followed by cation chromatography, Eshmuno®HCX was used as composite chromatography medium, the chromatography column was washed 3 times the column volume with an equilibration solution (10mM acetic acid-sodium acetate buffer, pH 5.0). The filtrate was directly loaded to the column with a sample volume of 80 g/L, then the column was washed 5 times the column volume with a washing solution (10mM acetic acid-sodium acetate buffer, pH 6.0) to remove portion of the pigment and HCP. Finally, an eluent (30 mM Tris-HCl, pH 8.5) was used for elution and the elution peak was collected to obtain insulin precursor with a purity of 90.1% and a yield of 93%. The results are shown in Figure 3. When detecting the host cell protein and exogenous DNA in the elution peak, it was found that the removal rate of the host cell protein and the exogenous DNA reached 91.0% and 89.0% respectively after one step of chromatography of the filtrate. The results are shown in Table 1.

**Table 1 Detection results and removal rate of HCP and HCD in precursor capture sample**

| Corresponding example | Sample name | HCP removal rate | HCD removal rate |
|---|---|---|---|
| Example 9 | Capture liquid | 95.6% | 89.6% |
| Example 10 | Capture liquid | 96.8% | 89.8% |
| Example 11 | Capture liquid | 91.0% | 89.0% |

| | | | |
|---|---|---|---|
| Note: The detection method of HCP is General Rule 3414 of Volume VI of the Chinese Pharmacopoeia, 2015 edition, and commercial kits are used for detection. HCD was detected by real-time fluorescent quantitative PCR method (q-PCR method) based on SYBR Green fluorescent dye. | | | |

It is to be understood for those skilled in the art that the foregoing description of specific examples is purely illustrative, and that various changes and modifications can be made to these examples without departing from the principle and essence of the present invention. Hence the protection scope of the present invention is defined by the appended claims.

## Claims

1. A method for preparing a precursor of recombinant human insulin or an analogue thereof comprising the following steps:
a. performing continuous flow centrifugation on obtained fermentation broth after the fermention of bacteria, and collecting a light liquid;
b. filtering the light liquid in step a by hollow fiber membrane filtration and collecting obtained filtrate;
c. purifying the filtrate in the step b by chromatography column.

2. The method of claim 1, wherein the hollow fiber membrane preferably has a pore size of 0.22 µm or 0.45 µm, more preferably 0.22 µm; or, the hollow fiber membrane has a molecular weight cut-off of 500-1000 KDa, preferably 750 KDa.

3. The method of claim 1 or 2, wherein the hollow fiber membrane filtration in step b is a cyclical tangential flow filtration.

4. The method of claim 1, comprising the following steps:
a. performing continuous flow centrifugation on obtained fermentation broth after the fermention of bacteria, and collecting a light liquid and a heavy liquid respectively; diluting the heavy liquid at least once and then performing centrifugation again to obtain another light liquid, and pooling two obtained light liquids into one;
b. filtering the light liquid obtained in step a by a cyclical tangential flow filtration using a hollow fiber membrane and collecting the filtrate;
c. purifying the filtrate in step b by a chromatography column using a composite filler as filler, which comprises a cation exchange ligand and a hydrophobic ligand, and the preferred cation exchange ligand is a strong cation exchange ligand.

5. The method of claim 4, wherein the diluting in step a adopts a diluent that is selected from an acidic solution or an alkaline solution, wherein the acidic solution is selected from an acetic acid-sodium acetate buffer and a citrate buffer, and the alkaline solution is selected from a trihydroxymethyl-aminomethane-hydrochloric acid buffer, a phosphate buffer and a glycine sodium hydroxide buffer.

6. The method of claim 5, wherein the acidic solution has a pH of 2.0-6.0, and the alkaline solution has a pH of 7.0-9.0.

7. The method of claim 5 or 6, wherein the acidic solution has a concentration of 1 mM-100 mM, preferably 1 mM-50 mM, more preferably 10 mM-20 mM, most preferably 10 mM; the alkaline solution has a concentration of 1 mM-100 mM , preferably 1mM-50mM, more preferably 10mM-30mM, most preferably 20mM.

8. The method of any one of claims 1-7, wherein the fermentation broth in step a is adjusted to a pH of 2.0-9.0, preferably a pH of 3.0-8.5, more preferably a pH of 4.0-8.0 before centrifugation.

9. The method of claim 8, wherein the pH of the fermentation broth in step a is adjusted to about 2.0, 2.5, 3.0, 3.5, 4.0, 4.5, 5.0, 5.5, 6.0, 6.5, 7.0, 7.5, 8.0 or 8.5 before centrifugation.

10. The method of claims 1-4, wherein the continuous flow centrifugation in step a is performed by a disc centrifuge and preferably selects a centrifugal force of 8000-14000g.

11. The method of claim 1, comprising following steps:
a. adjusting the fermentation broth to a pH of 3.0-8.0 after the fermention of bacteria, performing continuous flow centrifugation and collecting a light liquid;
b. filtering the light liquid in step a by a cyclical tangential flow filtration system using a hollow fiber membrane and collecting the filtrate;
c. purifying the filtrate in step b by chromatography column using a strong cation-hydrophobic exchange ligand;
wherein, step a comprises following steps: collecting light liquid and heavy liquid, respectively, diluting the heavy liquid at least once and then performing centrifugation to obtain another light liquid, and pooling two light liquids into one; the diluting adopts a diluent that is selected from an acetic acid-sodium acetate buffer with a pH of 3.0-5.0 or a trihydroxymethyl-aminomethane-hydrochloric acid buffer with a pH of 7.0-9.0;
the hollow fiber membrane in step b has a pore size of 0.22 µm.

12. The method of claims 1-11, wherein the purifying in step c comprises steps of equilibration, loading, washing impurities and elution; wherein the equilibration and washing use a solution of acetic acid-sodium acetate buffer, and the elution uses a solution of trihydroxymethyl-aminomethane-hydrochloric acid buffer.

13. The method of claim 12, wherein the acetic acid-sodium acetate buffer has a concentration of 1-50 mM, preferably 10 mM; the trihydroxymethyl-aminomethane-hydrochloric acid buffer has a concentration of 10 mM-150 mM, preferably 30 mM-100 mM.

14. The method of claim 12 or 13, wherein the solution used for the equilibration has a pH of 3.0-6.0, preferably 4.0-5.0; the solution used for washing impurities has a pH of 5.0-7.0, preferably 5.5-6.0.

15. A method for preparing a human recombinant insulin or an analogue thereof comprising:
1) expressing a precursor of human recombinant insulin or an analogue thereof by yeast;
2) purifying the precursor of human recombinant insulin or the analogue thereof according to the method of any one of claims 1-14;
3) performing enzymatic digestion on the precursor of human recombinant insulin or the analogue thereof to obtain the human recombinant insulin or the analogue thereof;
wherein the human recombinant insulin analogue is preferably a human insulin with B30 deletion.

16. A method for preparing an acylated insulin analogue, wherein the method comprising:
1) expressing a precursor of human recombinant insulin by yeast;
2) purifying the precursor of human recombinant insulin according to the method of any one of claims 1-14;
3) performing enzymatic digestion on obtained human recombinant insulin;
4) conducting a substitution of an acylation group on the human recombinant insulin;
wherein the substitution is preferably a substitution of lysine at position B29.

17. The method of claim 16, wherein the acylated insulin analogue is a human recombinant insulin with B30 deletion, preferably the substitution has a product of Lysine B29 (N^{ε}-(N^{α}-hexadecanedioic acid-L-lysine-N^{ε}-oxobutanoyl)) des(B30) human recombinant insulin.
